# EUROPÄISCHE PATENTSCHRIFT

(11) **EP 1 371 644 B1**
(45) Veröffentlichungstag und Bekanntmachung des Hinweises auf die Patenterteilung: **08.03.2006**
(21) Anmeldenummer: 03013075.1
(22) Anmeldetag: 10.06.2003
(51) Int. Cl.: C07D 201/12

(54) **Verfahren zur Herstellung von Caprolactam aus Polyamid-haltigen Abfällen**
Process for the preparation of caprolactam from polyamide containing waste
Procédé pour la préparation de caprolactam à partir de dechets contenant des polyamides

(30) Priorität: 10.06.2002 DE 10225692
(43) Veröffentlichungstag der Anmeldung: 17.12.2003
(73) Patentinhaber: Zimmer Aktiengesellschaft, 60388 Frankfurt am Main (DE)
(72) Erfinder: Born, Claus, 55218 Ingelheim (DE); Kämpf, Rudolf, Dr., 63584 Gründau (DE); Seelig, Joachim, Dr., 63599 Biebergemünd (DE); Wolf, Reinhard, 63517 Rodenbach (DE)
(74) Vertreter: Grünecker, Kinkeldey, Stockmair & Schwanhäusser Anwaltssozietät

(56) Entgegenhaltungen:
- IN-A- 142 150
- US-A- 5 359 062
- US-A- 5 656 757
- US-A- 5 990 306
- US-A- 6 111 099
- PATENT ABSTRACTS OF JAPAN Bd. 2000, Nr. 05, 14. September 2000 (2000-09-14) & JP 2000 038377 A (TORAY IND INC), 8. Februar 2000 (2000-02-08)

## Beschreibung

Die vorliegende Erfindung betrifft ein kontinuierliches und wirtschaftliches Verfahren zur Herstellung von hochreinem und für die Polykondensation von Polyamid 6 (Polycaprolactam) geeignetem Caprolactam aus Polyamidabfällen.

Bei sauberen, unkonfektionierten, fabrikneuen Polyamiden ist eine werkstoffliche Aufbereitung zu Produkten mit Qualitätsmerkmalen, wie sie Neuware aufweist, problemlos möglich. Sind dagegen Gebrauchsartikel, die schon jahrelang benutzt wurden oder Produkte aus einer Kunststoffsammlung wiederzuverwenden, lässt sich dies nur mit hohem technischen, energetischen und kostenmäßigem Aufwand durchführen.

Dabei sind Reinigungs- und Sortiervorgänge notwendig um sortenreines Gut zu erhalten, da Kunststoffprodukte immer dem Einsatzgebiet angepasst werden und enthalten daher hersteller- und herstellungsbedingt Zuschlagsstoffe, Farbmittel, Stabilisatoren, Glasfasem, etc., die eine Aufbereitung erschweren. Daher führt bei einer wirtschaftlichen Wiederverwendung von Polyamidabfällen kein Weg an der Zerlegung in die Monomere vorbei, soll ein Produkt erzeugt werden, das sich von Ware, die aus Monomeren der Syntheseroute hergestellt wurde, nicht unterscheidet.

Caprolactam ist das Monomer das benutzt wird, um Polyamid 6 herzustellen, aus dem dann Teile für Fahrzeuge und Spritzgussteile mit Glasfasern und anderen Zuschlägen hergestellt werden sowie Fasern für Garne gesponnen werden, aus denen wiederum Teppiche, Teppichbodenbeläge oder andere Gegenstände des täglichen Lebens gefertigt werden.

Ein großer Teil dieses Materials wird nach dem bestimmungsgemäßen Gebrauch in den Kunststoffkreislauf zugeführt und geht über das Kunststoffrecycling zur Wiederverwertung. Da die Polyamid 6 Produkte meist nicht das einzige Material sind, das amidische Gruppen enthält, sondern auch noch Polyamide zugegen sind, die aus unterschiedlichen Disäuren und Diaminen bestehen, muss eine Kennung und Sortierung in die einzelnen Polyamid-Produktgruppen erfolgen. Erfolgt eine solche Trennung nicht, dann ist mit erheblichen Schwierigkeiten durch Mischreaktionen der verschiedenen Monomergruppen, Ausbeuteverlusten durch Nebenprodukte und ungenügender Qualität bei dem erhaltenen Monomer zu rechnen. Ein Grossteil des Polyamid 6 Kunststoffes geht beispielsweise in die Herstellung von Fasern und hier speziell in die Anwendung für Bodenbeläge. Das Polyamid 6 wird zu Fasern versponnen, die in Teppichfabriken auf ein Trägergewebe, z.B. Polypropylenvlies, genadelt und je nach Teppichart zu Schlingen- oder Veloursware verarbeitet werden. Nach der Nadelung des von Spulen abgezogenen Garnes auf dem Träger erfolgt eine Fixierung der Polfasem durch eine Klebeschicht auf die anschließend ein Polymerschaumrücken aufgetragen wird, der mit Füllstoffen, wie Kreide, gefüllt ist. Der Schaumrücken erfüllt zum einen den Zweck, dem Teppich eine gute Trittschalldämmung zu verleihen und zum anderen das notwendige Gewicht zu erzeugen damit er plan ohne Verklebung auf dem Boden aufliegt.

Die Aufbereitung von Polyamid 6 Abfällen nach ihrem bestimmungsgemäßen Gebrauch wurde in verschiedenen Patenten, wie beispielsweise DE 19719734, EP 0681896, US 5598980, DE 2416573, und DE 2507744 beschrieben, und umfasst die Erkennung und Sortierung von Nichtpolyamid 6―Ware, deren Zerkleinerung und die Abscheidung von Schmutz- und Fremdstoffbeigaben. US 5656757 beschreibt ein Verfahren, in dem ein Destillationsrückstand in den Depolymerisator zurückgeführt wird. In JP 2000 03877 wird Caprolactam nach der Depolymerisationsstufe zurückgeführt. In IN 142150 wird die Mutterlauge der Kristallisation in die Destillation rückgeführt. Für die Trennung von einzelnen Komponenten, wie sie beispielsweise bei der Zerkleinerung von Teppichen in Form von Schaumrücken, Polfaser und Träger anfallen, werden bevorzugt mehrstufige Schwimm-/Sinkverfahren benutzt, in deren Folge mittels Zentrifugen und Salzlösungen unterschiedlicher Dichte ein Aufschwimmen der leichteren

Fraktion, wie Polypropylengewebe, und ein Absinken von schwererem gefülltem Teppichrücken bewirkt wird. Eine nach diesem Verfahren gewonnene Polyamid 6 Fraktion wird durch Waschen von anhängender Salzfracht befreit und in einem kontinuierlichen Verfahren getrocknet und über einen Extruder der Depolymerisation zugeführt. In der Depolymerisation erfolgt beispielsweise nach den aus DE 887199, EP 0875504, DE 910056, US 4605762, und JP 53-13636 bekannten Verfahren mittels Phosphorsäure eine Spaltung der Polymerketten und eingeleiteter Dampf treibt das entstandene Caprolactam als Wasserdampfgemisch ab.

Weiterhin ist aus US 5990306 ein Verfahren zur Herstellung von gereinigtem Caprolactam aus einem Polyamid-haltigen Teppich bekannt, bei dem nach der Depolymerisation das Caprolactam destilliert und kristallisiert wird.

Außerdem ist aus US 4107160 ein Verfahren zur kontinuierlichen Rückgewinnung von Caprolactam aus Polycaprolactam-Abfällen bekannt, wobei unter anderem ein Caprolactam-Wasser-Gemisch, das bei der Destillation des Caprolactams erhalten wird, mit frischem Wasserdampf gemischt wird und als überhitztes Gemisch der Depolymerisation zugeführt wird.

Die Gewinnung von hochreinem Caprolactam aus Polyamid 6 Abfällen, wie sie beispielsweise aus gebrauchten Teppichen oder den anderen oben erwähnten Produkten erfolgt, unterliegt ständigen Schwankungen der Zusammensetzung des Eingangsstoffstromes. So sind einerseits die Nebenbestandteile eines Teppichs wie Schaumrücken oder Trägergewebe je nach Hersteller verschieden und andererseits das Polyamid 6 unterschiedlich mit Additiven, Farbstoffen, etc. versetzt. Es herrscht daher eine nicht einheitliche und kontinuierliche Rohstoffversorgung vor. Aus einem Material mit solch mitunter extrem schwankenden Eingangsprodukten ein hochreines Produkt herzustellen, das die Qualitätsanforderungen des synthetisiertem Caprolactam erfüllt oder übertrifft, ist großtechnisch nicht erreicht worden.

Nachteilig an den bekannten Verfahren zur Aufbereitung von Polyamid 6 Abfällen ist es daher, dass diese kein hochreines Caprolactam, das zur Herstellung von Polyamid 6 verwendbar ist, liefern.

Aufgabe der vorliegenden Erfindung ist es daher, ein Verfahren zur Herstellung von Caprolactam aus Polyamid-haltigen Abfällen zur Verfügung zu stellen, das kostengünstig und einfach durchzuführen ist und mit dem ein hochreines und zur Polykondensation zu einem hochwertigen und von einem aus industriellem Caprolactam hergestellten Polyamid 6 nicht zu unterscheidendem Produkt geeignetes Caprolactam herstellbar ist.

Die Lösung der Aufgabe ist ein Verfahren zur Herstellung von Caprolactam aus Polyamid-haltigen Abfällen, umfassend die Schritte
a) Depolymerisieren der Polyamid-haltigen Abfälle, wodurch ein Caprolactam-Rohmaterial und gegebenenfalls ein Nebenbestandteile bzw. Zuschlagsstoffe enthaltender Strom erhalten wird,
b) wenigstens einmaliges Destillieren des Caprolactam-Rohmaterials, und
c) wenigstens einmaliges Kristallisieren des in Stufe b) erhaltenen Caprolactam-Materials, wodurch Caprolactam erhalten wird,

wobei wenigstens ein Teil des in Stufe c) erhaltenen Caprolactams mit einer Permanganatzahl von < 10000 sec. und einer UV-Transmission von < 85 % den Polyamid-haltigen Abfällen vor und/oder während der Depolymerisation zugesetzt wird.

Insbesondere bevorzugt wird das erfindungsgemäße Verfahren kontinuierlich durchgeführt.

Das Polyamid-haltige Material ist vorzugsweise ausgewählt aus der Gruppe, bestehend aus Polyamid-haltigen Formkörpern, wie Teilen für Fahrzeuge, Polyamid-haltigen Spritzgussteilen mit Glasfasern und anderen Zuschlägen sowie Polyamid-haltigen Fasern, Teppichen, Teppichbodenbelägen und anderen Polyamid-haltigen Gegenständen des täglichen Lebens, wie Kleidung. Das Polyamid-haltige Material, das der Depolymerisation zugeführt wird, kann außerdem Nichtpolymere und artfremde Zuschlagsstoffe enthalten.

Die Polyamid-haltigen Abfälle werden vor dem Durchführen der Depolymerisation vorzugsweise zunächst sortiert, so dass diese überwiegend Polyamid 6 enthalten und die übrigen anderen Stoffe abgetrennt werden, wie vorstehend beschrieben.

Außerdem werden die Polyamid-haltigen Abfälle vorzugsweise vor dem Depolymerisieren verdichtet und aufgeschmolzen.

Die Depolymerisation wird vorzugsweise bei einer Temperatur von etwa 180 bis etwa 300 °C und Drücken von etwa 0,5 bis etwa 2 bar durchgeführt, wie in DE 887199, EP 0 875 504, DE 910056, US 4605762, US 5990306 und JP 53-13636 beschrieben. Außerdem wird bevorzugt ein basischer oder saurer Katalysator, wie Phosphorsäure, zugesetzt. Die Reaktionsbedingungen während der Depolymerisation werden bevorzugt der sich wandelnden Zusammensetzung des dem Verfahren zugeführten Rohstoffes, nämlich des Polyamid-haltigen Abfalls, angepasst. So kann die Dampftemperatur erhöht, größere Dampfmengen verwendet, das Molverhältnis von Phosphorsäure zu Polyamid verändert und/oder der Druck erhöht oder emiedrigt werden.

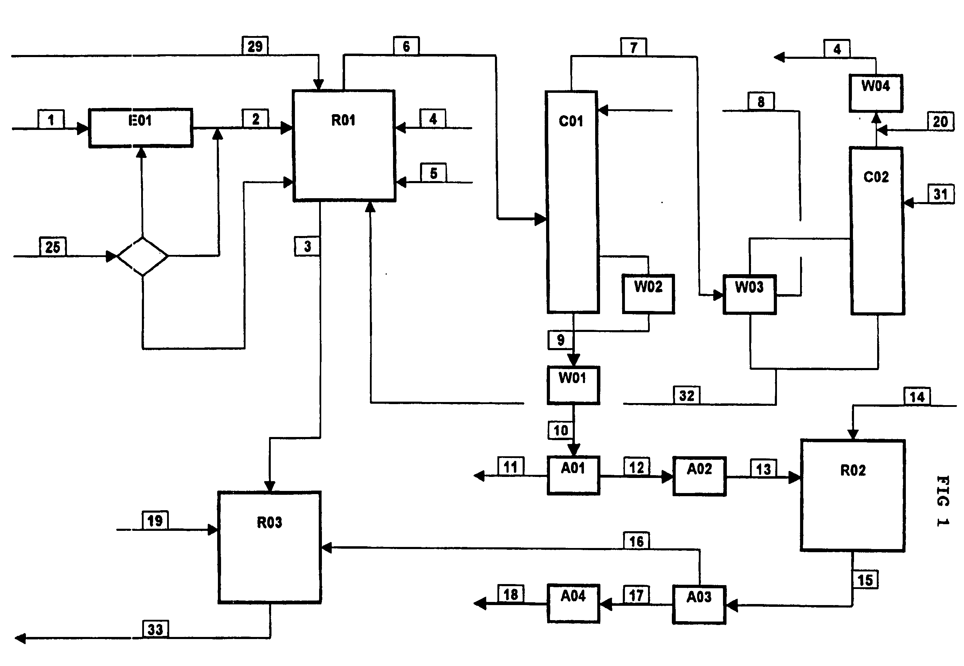

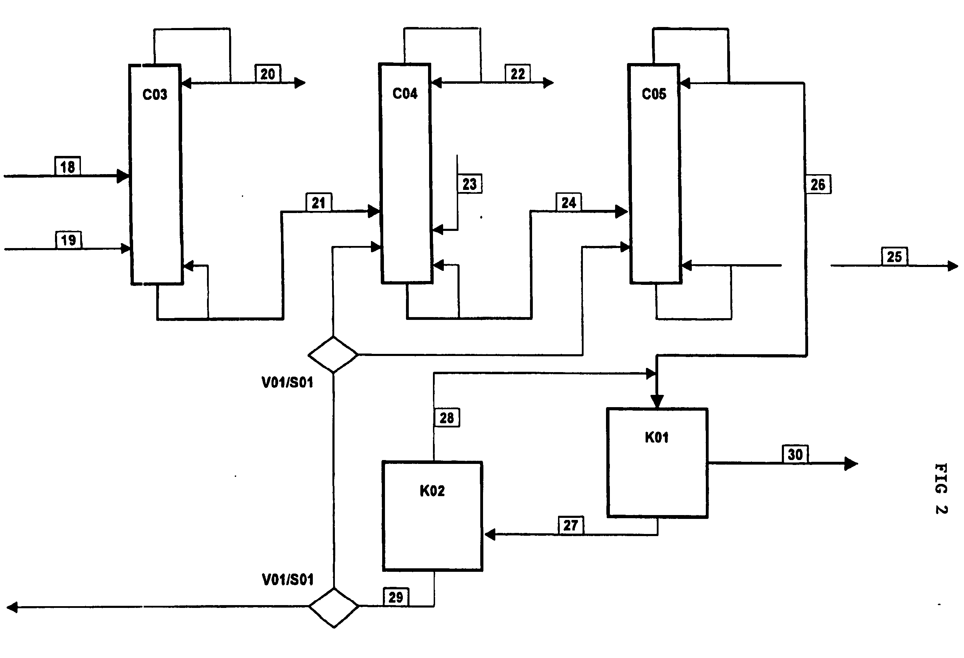

Nach dem Depolymerisieren wird im erfindungsgemäßen Verfahren wenigstens eine Destillation durchgeführt, wie auch anhand der Fig. 1 und 2 beschrieben. Die Begriffe "Destillieren" und "Destillation" werden hier allgemein verwendet für Destillieren, fraktioniertes Destillieren und Reinigungsschritte, wie sie in Kolonnen bzw. Einrichtungen, beispielsweise in Vakkumkolonnen, Extraktionskolonnen, Eindickungskolonnen, Rektifikationskolonnen und Refraktionskolonnen (siehe Fig. 1 und 2), durchgeführt werden.

Es werden vorzugsweise wenigstens vier Destillationen durchgeführt, wobei bei der ersten Destillation, wie in Apparatur C01, eine Aufkonzentration des Rohlactam/Wasser Gemisches stattfindet. Nach Durchlaufen mehrerer Abscheide- und Raffinationsstufen gelangt das Rohcaprolactam Konzentrat in eine zweite Destillation, wie in die Eindampfeinrichtung C03, wo Wasser abgetrieben wird und eine weitere Aufkonzentrierung stattfindet. Hier erfolgt durch Zugabe von Lauge eine Neutralisation acider Anteile. Die dritte Destillation, wie die Destillationseinrichtung C04, hat die Aufgabe niedriger als Caprolactam siedende Bestandteile unter Zugabe von ammoniakhaltigem Stickstoff abzustrippen. Nach Verlassen der dritten Destillation, wie in der Destillationseinrichtung C04, gelangt das Rohcaprolactam in die vierte Destillation, wie in die Destillationseinrichtung C05, wo unter reduziertem Druck ein destilliertes reines Caprolactam über Kopf abgezogen wird. Da dies jedoch noch nicht den Kriterien für ein hochreines Produkt genügt, wird es der wenigstens einen Kristallisation, wie der fraktionierten Kristallisation K01/K02, zugeführt.

Außerdem kann das aus der Depolymerisation in der Dampfphase entweichende Spaltcaprolactam-Wasser-Gemisch einer ersten Destillation (Eindampfung) zugeführt werden, bei der die entweichenden und energiereichen Brüden benutzt werden können, das aus der Polyamid 6 Granulat Extraktion anfallende und noch Monomere und Oligomere enthaltende Wasser zu erhitzen und zu verdampfen, wobei die energiereichen Brüden kondensiert und ais Rücklauf zur Eindampfung zurückgeführt werden. Die Brüden aus der ersten Caprolactamdestillation, die noch Caprolactam gemäss dem Dampfdruckgleichgewicht enthalten und nur teilweise kondensiert werden, können so genutzt werden. Die Teilkondensation in Höhe der notwendigen Menge des Rücklaufverhältnisses und die Nutzung des Energieinhaltes der restlichen Brüden in der Depolymerisation ist vorteilhaft. Außerdem kann der Dampf aus der Polyamid-Extraktion-Konzentration, wie in Einrichtung C05, in die Depolymerisation zurückgeführt.

Die aus der Extraktwassereindampfung C02 und der Rohcaprolactamdestillation C03 entweichenden Brüden können außerdem vorteilhaft einem Überhitzer zugeführt werden. Der dabei entstehende überhitzte Dampf kann zur Spaltung der Polymerketten der Depolymerisation zugeführt werden.

Insbesondere hat es sich als wirtschaftlich vorteilhaft erwiesen, einen Destillationsrückstand vor und/oder in die Depolymerisation rückzuführen, der aus einer im Herstellungsverfahren des Caprolactams aus Polyamid-haltigen Abfällen der Depolymerisation nachgeschalteten (Vakuumkolonne) Destillationseinrichtung zur Reinigung des Caprolactams stammt und der neben kurzkettigen Polymeren, Farbträgern und Produkten aus Nebenreaktionen noch verwertbare Mengen an Caprolactam enthält, wie nachstehend auch anhand der Fig. 1 und 2 beschrieben.

Dieses Vorgehen hat sich dann als besonders vorteilhaft erwiesen, wenn der Depolymerisation ein Aufschmelzen des Polyamid-haligen Materials voraus geht und das Aufschmelzen der Polyamid-haltigen Abfälle unter Vakuum stattfindet. Eine weiteres Verfahren hat sich dann im Hinblick auf den Polymerabbau und den Energieverbrauch als gegenüber dem vorhergehend beschriebenen als wirtschaftlich stärker vorteilhaft bewährt, wenn die Zuführung eines Rückstandes aus einer Vakuumkolonne (Destillatioriseinrichtung zur Reinigung des Caprolactams) während des Aufschmelzens so an einer Steile in einen Extruder zum Aufschmeizen erfolgt, dass die ins Vakuum gehenden Brüden nicht belastet werden. Dies kann beispielsweise in der Verdichtungszone erfolgen. Der eingespeiste Rückstand aus der Vakuumkolonne setzt zum einen die Schmelzeviskosität herunter, fördert zum anderen den Abbau der Polymerketten und erleichtert so den Transport durch den Extruder bei niedrigerer Antriebskraft.

Vorzugsweise werden im erfindungsgemäßen Verfahren wenigstens zwei Kristallisationsschritte durchgeführt, wobei beide Schritte bevorzugt fraktioniert durchgeführt werden. Dabei scheidet sich das gewünschte Produkt, nämlich hochreines Caprolactam, zuerst ab wird und nach einer Analyse entweder bis zur Herstellung von Polyamid daraus gelagert oder einer nochmaligen Depolymerisation, Destillation und/oder Kristallisation zugeführt, wie nachstehend anhand der Fig. 1 und 2 beschrieben.

Im erfindungsgemäßen Verfahren wird mindestens einmal ein fraktioniertes Kristallisations-Schmelzverfahren angewendet.

Es ist weiterhin vorteilhaft, dass das kristallisiertes Caprolactam, das aus der mindestens einen Kristallisation erhalten wird, vorzugsweise nach einer ersten Reinigungsstufe des fraktionierten Kristallisations-Schmelzverfahrens, zur Erreichung einer, einem synthetisierten Caprolactam mindestens gleichwertiger Qualität genügend, einer kontinuierlichen Analyse wie Permanganatzahl, UV-Transmission, APHA-Zahl, Alkalität und flüchtigen Basengehalt unterzogen wird.

Das nach Stufe c) erhaltene Caprolactam mit einer Permanganatzahl von < 10000 sec. und einer UV-Transmission von < 85 % (auch als rückgeführtes Caprolactam bezeichnet), bevorzugt mit einer Permanganatzahl von < 8000 sec. und einer UV-Transmission von < 82 %, das dem Polyamid-haltigen Material vor und/oder während der Depolymerisation zugesetzt wird, ist ein Caprolactam, das den Kriterien für ein hochreines und zur Produktion von Polyamid 6 Polykondensaten bester Qualität geeignetem Produkt nicht genügt. Die Rückführung dieses Caprolactams im erfindungsgemäßen Verfahren fördert unter anderem den Abbau des Polyamid-haltigen Materials.

Bevorzugt wird das gesamte nach Stufe c) erhaltene Caprolactam mit einer Permanganatzahl von < 10000 sec. und einer UV-Transmission von < 85 % vor und/oder während der Depolymerisation zugesetzt.

Zusätzlich bevorzugt kann wenigstens ein Teil des in Stufe c) erhaltenen Caprolactams mit einer Permanganatzahl von < 10000 sec. und einer UV-Transmission von < 85 %, vorzugsweise einer Permanganatzahl < 8000 sec. und einer UV-Transmission von < 82 %, in Stufe b) zugesetzt werden.

Insbesondere bevorzugt kann das aus der Stufe c), der Kristallisation, erhaltene Caprolactam in Abhängigkeit von dessen Reinheit entweder wieder der Depolymerisation, der Destillation oder nochmals der Kristallisations zugeführt werden.

Insbesondere bevorzugt wird Caprolactam dessen Qualitätsmerkmale APHA >5, UV-Transmission <80, Permanganatzahl <5000 sec, Alkalität >1mmol/kg, flüchtige Basen>1 mmol/kg aufweisen, vorteilhaft in die Depolymerisation zurückgefahren, Caprolactam mit Werten APHA >3, UV-Transmission <82%, Permanganatzahl <8000 sec, Alkalität ≥0,5 mmol/kg, flüchtige Basen ≥0,5 mmol/kg wird vorteilhaft einer der Destillationsstufen und Caprolactam mit APHA ≥4, UV-Transmission ≤85%, Permanganatzahl ≤10000 sec, Alkalität ≤0,5 mmol/kg, flüchtige Basen ≤0,5 mmol/kg wird bevorzugt der Kristallisation zugeführt.

Des weiteren ist es bevorzugt beim Durchführen des erfindungsgemäßen Verfahrens Nebenbestandteile und Zuschlagsstoffe des eingesetzten Polyamid-haltigen Materials, wie SBR-Schaumrücken, Kreide, Russ und Geweberückstände, nach der Depolymerisation kontinuierlich und durch Analyse und Überwachung des Spaltsäure- und Stickstoffgehaltes des Stromes der die Zuschlagsstoffe enthält kontrolliert und geregelt in Abhängigkeit vom Stickstoffgehalt und Katalysatorgehalt, beispielsweise Phosphorsäure, auszuschleusen.

Es hat sich als vorteilhaft erwiesen, den Phosphorsäuregehalt in der Ausschleusung zwischen 0.1 Massen % und 50 Massen%, besonders vorteilhaft zwischen 1 Massen% und 25 Massen % zu halten. Der Gehalt an Stickstoff liegt vorteilhaft zwischen 0.1 Massen % und 10 Massen%, besonders vorteilhaft zwischen 1 Massen% und 5 Massen %.

Insbesondere wird die Analyse und Überwachung des Spaltsäure- und Stickstoffgehaltes des Stromes, der die Zuschlagsstoffe und Nebenbestandteile enthält und die Depolymerisation verlässt, vorteilhaft kontinuierlich durchgeführt, da sich die Zusammensetzung des dem Verfahren zugeführten Rohstoffes, nämlich der Polyamid-haltigen Abfälle, beständig ändert,

Außerdem ist es bevorzugt, dass das Rohcaprolactamkonzentrat aus einer ersten Destillation (Eindampfung C 01) durch kontinuierliche Analysen auf seine Qualitätsmerkmale wie Permanganatzahl, UV-Transmission, APHA-Zahl, Alkalität und flüchtigen Basengehalt untersucht wird.

Vorzugsweise hat das Rohcaprolactam die folgenden Mindest - Eigenschaften: APHA >4, UV-Transmission <82, Permanganatzahl <8000 sec, Alkalität >0,5 mmol/kg, flüchtige Basen>0,5 mmol/kg. Sollten die Werte nicht den Analysen eines industriell auf dem Syntheseweg hergestellten und gewünschten Caprolactam entsprechen, ist es vorteilhaft, dass die Betriebsbedingungen in C01-C05, R02 und K01,K02 und die Qualitätsmerkmale wie Permanganatzahl, UV-Transmission, APHA-Zahl, Alkalität und flüchtigen Basengehalt einem Speicher zugeführt werden, durch Neuronale Netz Software Korrelationen von Betriebsbedingungen und Qualitätsmerkmalen bestimmt werden und diese zur Steuerung und Regelung der Destillation (Eindampfung) im wirtschaftlichsten Bereich benutzt werden. Dazu werden aus verknüpften linearen und nichtlinearen Gleichungsystemen höherer Ordnung, deren Konstanten durch Gleichungslöser ermittelt. Ein wesentlicher Punkt sind ausserdem Kennfelder in denen Abhängigkeiten der Produktqualität von den Prozessbedingungen erfasst werden, wie sie ähnlich aus der Automobilindustrie zur Motorsteuerung bekannt sind. Wesentliche Merkmale dieser Regelung sind, dass beispielsweise bei dem Unterschreiten der UV-Transmission die Dampftemperatur reduziert, bei einer Zunahme von Alkalität und flüchtigem Basengehalt die Phosphorsäurekonzentration erhöht und bei einer Zunahme der Permanganatzahl der Spaltdampf- bzw.Druck im Depolymerisationsreaktor verringert wird. In den Destillationskolonnen C01-C05 erfolgt in Abhängigkeit von der Abweichung der Kriterien die an ein hochreines Caprolactam zu stellen sind eine Reduzierung oder Erhöhung des Drucks. Eine Erhöhung des Rücklaufverhältnisses oder ein Absenken der Temperatur werden durchgeführt, wenn die Farbwerte schlechter werden.

Im erfindungsgemäßen Verfahren ist es bevorzugt in einem Raffinationsschritt dem nach Stufe b) erhaltenen Caprolactam Additive und/oder Oxidationsmittel zuzusetzen. Wenn in Stufe b) mehr als eine Destillation durchgeführt wird, dann können die Additive und/oder Oxidationsmittel dem nach der ersten und vor der letzten Destillation erhaltenen Caprolactam mindestens einmal zugesetzt werden. Vorzugsweise werden die Additive und/oder Oxidationsmittel nach der ersten Destillation zugesetzt.

Insbesondere bevorzugt ist es, dass das Rohcaprolactamkonzentrat aus der Destillation (Eindampfung C01) zur Verbesserung der Qualitätsmerkmale wie Permanganatzahl, UV-Transmission, APHA-Zahl, Alkalität und flüchtigen Basengehalt durch mindestens einen Abscheider vor und hinter der Destillation zur Behandlung mit Additiven und/oder Oxidationsmitteln geleitet wird, Dabei kann die Zugabe von Additiven und/oder Oxidationsmitteln zum Rohcaprolactamkonzentrat in einem Raffinationsschritt wegen der nicht einheitlichen Rohstoffzusetzung des Eingangsproduktes, nämlich der Polyamid-haltigen Abfälle, durch kontinuierliche Analyse überwacht und die zugeführte Menge gesteuert wird. Die Dosierung eines Oxidationsmittels, wie einer wässrigen Kaliumpermanganatlösung, erfolgt in Abhängigkeit von der UV-Transmission und der APHA-Zahl und ist so zu gestalten, dass bei schlechter werdender Farbe mehr Permanganat zugegeben wird.

Unterschreitet beispielsweise die UV-Transmission den Wert von 85% erfolgt die Zuspeisung eines Adsorptionsmittels wie beispielsweise Aktivkohle, unterhalb einer Grenze von 66% erfolgt die Zugabe eines weiteren Hilfsmittels wie beispielweise Diatomen Erde.

Diese Steuerung ist nicht konstant aber proportional den Veränderungen, da die mit dem Rohmaterial eingetragenen Störstoffe die zu dosiernde Menge bestimmen und eine konstante Produktqualität nur so gewährleistet werden kann, weshalb die Produkteigenschaften gemessen werden. Dabei kann erreicht werden, dass mit möglichst wenig Additiv der größtmögliche Effekt erzielt wird und eine Überdosierung oder Unterdosierung als Folge der schwankenden Rohmaterialzusammensetzung vermieden werden kann, da dies wieder Kosten und Produktqualitätsverschlechterungen verursacht. In den nachfolgenden Reinigungsstufen können dann strengere/schärfere Betriebsbedingungen verwendet werden, um diese Poduktqualitätsverschlechterungen auszugleichen.

Außerdem kann vorzugsweise nach Stufe b) eine Festbettfiltration des erhaltenen Caprolactams durchgeführt werden. Wenn mehr als eine Destillation in Stufe b) durchgeführt wird kann eine Festbettfiltration nach mindestens einer Destillation durchgeführt werden. Dabei wird die Festbettfiltration insbesondere bevorzugt nach der ersten Destillation durchgeführt. Wenn dem Caprolactam nach oder während der Stufe b) Additive und/oder Oxidationsmittel zugesetzt werden kann eine Festbettfiltration vor und/oder nach deren Zugabe durchgeführt werden.

Zur wirtschaftlichen Nutzung von Abwärmen und der Verringerung des Verbrauchs an Wasser, können die Brüden aus einer ersten oder zweiten Destillation (Eindampfung C 03) nur teilweise und in der Menge kondensiert werden, so dass die Rücklaufmenge gewährleistet ist, und die übrigen nicht kondensierten und heißen Dämpfe nach Überhitzung als Spaltdampf bei der Depolymerisation genutzt werden können.

Außerdem ist es bevorzugt bei der wenigstens einen Destillation (Eindampfung) Stickstoff mit einer Ammoniakkonzentration von vorteilhaft 100 ppm bis 5%, besonders vorteilhaft 250 ppm bis 3% einzuspeisen. Wenn mehr als eine Destillation durchgeführt werden, ist es bevorzugt Stickstoff in der dritte Destillation (Eindampfung C04) einzuleiten.

Außerdem ist es bevorzugt, dass in einer Destillation, insbesondere einer vierten Destillation (Caprolactamrektifikation C05), im Sumpf anfallende Oligomere mit niedrigem Molekulargewicht, die sich mit wirtschaftlichem Vorteil noch zu Caprolactam spalten lassen und sich vorteilhaft auf die Ausbeute auswirken, zur erneuten Aufbereitung dem Aufschmelzen oder der Depolymerisation zugeführt werden.

Außerdem kann der aus der Depolymerisation ausgeschleuste flüssige Strom anorganische Salze, überschüssige Phosphorsäure, artfremde Polymere und Oligomere, die nicht mehr zu Caprolactam gespalten werden können, mit den Rückständen aus einem Raffinationsreaktor, wie R02, vermischt werden und einer Neutralisation zugeführt werden, wie anhand der Fig. 1 und 2 nachstehend beschrieben. Der Rückstand aus der Neutralisation, der noch organische Bestandteile enthält, kann dann einer thermischen Verwertung zugeführt wird.

Durch das Durchführen des erfindungsgemäßen Verfahrens wird hochreines Caprolactam erhalten. Durch die Rückführung der schlechten Produktströme werden erstens eine hohe Ausbeute und zweitens ein hochreines Produkt erzeugt.

Ein weiterer Vorteil des erfindungsgemäßen Verfahren in einer vorstehend beschriebenen bevorzugten Ausführungsform, dass kein Frischdampf benötigt wird, wenn alles Wasser zur Dampferzeugung bzw. aller Dampf in der Spaltung innerhalb des Verfahrens recycliert wird.

In einer weiteren bevorzugten vorstehend beschriebenen Ausführungsform hat das erfindungsgemäße Verfahren den Vorteil, dass kein Abfallstrom die Anlage verlässt, der ein Caprolactam-Wasser-Gemisch enthält. So enthält der in den Fig.1 beschriebene Strom 33 geschmolzenes Polyamid/Phosphorsäure/Kreide/Zuschlagsstoffe aber kein Caprolactam. Alle Caprolactam-haltigen Ströme können intern rückgeführt und wieder aufbereitet werden.

Die Fig. 1 und 2 zeigen schematisch eine Analge zum Durchführen einer bevorzugten Ausführungsform des erfindungsgemäßen Verfahrens.

In den Fig. 1 und 2 bedeuteten:

| Ausrüstung | |
|---|---|
| A01 | Schwerkraftabscheider |
| A02 | Festbettfilter |
| A03 | Filter |
| A04 | Festbettfilter |
| C01 | Aufkonzentrierungseinrichtung |
| C02 | Eindampfeinrichtung |
| C03 | Destillationseinrirhtung |
| C04 | Destillationseinrichtung |
| C05 | Destillationseinrichtung |
| E01 | Aufschmelzeinrichtung |
| K01 | Kristallisationseinrichtung |
| K02 | Kristallisationseinrichtung |
| R01 | Depolymerisationreaktor |
| R02 | Raffinationsreaktor |
| R03 | Neutralisationsstufe |
| W01 | Kühler |
| W02 | Verdampfer |
| W03 | Verdampfer |
| W04 | Überhitzer |
| V01/S01 | Analysen gesteuerter Verteiler |

| Stoffströme | |
|---|---|
| 1 | Polyamid 6 Abfall |
| 2 | Schmelze |
| 3 | Auschleusung Depolymerisation |
| 4 | Dampf Poyalmid 6 Extraktion/Konzentration |
| 5 | Phosphorsäure |
| 6 | Rohcaprolactam/Wasser |
| 7 | Dampf |
| 8 | Kondensat |
| 9 | Rohcaprolactam Konzentrat |
| 10 | Rohcaprolactam Konzentrat |
| 11 | Öle |
| 12 | Rohcaprolactam Konzentrat |
| 13 | gefiltertes Rohcaprolactam Konzentrat |
| 14 | Additive |
| 15 | Additive + Rohcaprolactam Konzentrat |
| 16 | abgeschiedenes Additiv |
| 17 | Rohcaprolactam Konzentrat |
| 18 | gefiltertes Rohcaprolactam Konzentrat |
| 19 | Lauge |
| 20 | Wasserdampfrückführung |
| 21 | Rohcaprolactam Konzentrat |
| 22 | Leichtsieder |
| 23 | Stickstoff-Ammoniak Stripgas |
| 24 | Rohcaprolactam Konzentrat |
| 25 | Rohcaprolactam Rückstand |
| 26 | destilliertes, reines Caprolactam |
| 27 | Vorprodukt Caprolactam |
| 28 | fraktioniertes Caprolactam |
| 29 | Caprolactam Rückstand |
| 30 | hochreines Caprolactam |
| 31 | Polyamid 6 Extrakt |
| 32 | Polyamid-Extakt-Konzentrat |
| 33 | neutralisierte Ausschleusung |

Das erfindungsgemäße Verfahren wird nunmehr anhand der Fig. 1 und 2 näher erläutert, wobei die einzelnen Merkmale der Ausführungsform so verstanden werden sollen, dass diese unabhängig voneinander vorteilhaft im erfindungsgemäßen Verfahren angewendet werden können.

Die Herstellung des hochreinen und zur Polykondensation von Polyamid 6 Produkten allerbester Farb- und Produktqualität, die frei ist von eigenschafts- oder gebrauchsmindernden Nebenprodukten ist dadurch gekennzeichnet, dass die durch mechanische Verfahren zurückgewonnen Polyamid 6 Abfälle wie Abbildung 1 zeigt, über die Zuführung 1 einem Aufschmelzextruder E01 zugeführt werden. In diesem Extruder werden unter schonenden thermischen und mechanischen Bedingungen die Polyamid 6 Abfälle verdichtet und aufgeschmolzen. Es hat sich im Laufe des Betriebes einer Produktionsanlage als wirtschaftlich vorteilhaft erwiesen, dass Rückstand 25 der aus der Vakuumkolonne C05 stammt und der neben kurzkettigen Polymeren, Farbträgem und Produkten aus Nebenreaktionen noch verwertbare Mengen an Caprolactam enthält, nicht einer Entsorgung, sei es durch thermische Verwertung oder andere Verfahren, zugeführt wird, sondern als wertvoller Rohstoff zur Steigerung der Ausbeute zu dem eingehenden verunreinigten Material zurückgeführt und mit diesem vereinigt dem in die Depolymerisation gehenden Stoffstrom 2 zugesetzt wird. Die Einspeisung des Rückstandes 25 aus der Vakuumkolonne C05 kann hinter einem Aufschmelzextruder E01 in die Zuführungsleitung 2 oder in den Depolymerisationsreaktor R01 direkt erfolgen. Dieses Vorgehen hat sich dann als besonders vorteilhaft erwiesen, wenn das Aufschmelzen der Polyamidabfälle unter Vakuum stattfindet. Eine anderes Verfahren hat sich dann im Hinblick auf den Polymerabbau und den Energieverbrauch als gegenüber dem vorhergehend beschriebenen als wirtschaftlich stärker vorteilhaft bewährt, wenn die Zuführung des Destillationsrückstandes 25 aus der Vakuumkolonne CO5 während des Aufschmelzens so an einer Stelle in den Extruder E01 erfolgt, dass die ins Vakuum gehenden Brüden nicht belastet werden, dies kann beispielsweise in der Verdichtungszone erfolgen. Der eingespeiste Destillationsrückstand 25 aus der Vakuumkolonne C05 setzt zum einen die Schmelzeviskosität herunter, fördert zum anderen den Abbau der Polymerketten und erleichtert so den Transport durch den Extruder bei niedrigerer Antriebskraft.

Die den Aufschmelzapparat E01 verlassende Polyamidschmelze wird über die Zuleitung 2 dem Depolymerisationsreaktor R01 kontinuierlich zugeführt, in dem nach dem bekannten Verfahren nach DE 887199, EP 0875504, DE 910056, US 4605762, JP 53-13636 bei Temperaturen zwischen 180°C und 300°C, Drücken von 0,5 bar und 2 bar Phosphorsäure 5 und Dampf 4 zugespeist wird, wobei eine Aufspaltung der Polymerketten in Oligomere und Monomere erfolgt. Wie der einschlägigen Literatur beispielweise Brandrup/Bittner/Michaeli/Menges "Die Wiederverwertung von Kunststoffen", S. 513-520 Verlag Carl Hanser München Wien 1995 zu entnehmen ist, wird dabei wieder Caprolactam zurückgebildet, das zusammen mit dem Wasserdampf und weiteren dampfflüchtigen Bestandteilen über die Brüdenleitung 6 den Reaktor R01 verlässt.

Bei der Herstellung von Polyamid 6 Granulat durch ringöffnende Polykondensation von Caprolactam verbleiben noch Monomer und niedermolekulare Bestandteile als Rückstände im Polyamid 6 Granulat und verschlechtern die Gebrauchseigenschaften. Durch eine dem Fachmann bekannte Wasserextraktion werden diese Produkte dem Polymer entzogen. Die in dem Extraktwasser enthaltenen niedermolekularen Anteile und löslichen Monomere liegen in starker Verdünnung vor und verursachen bei einer Entsorgung als Abfall hohe Kosten. Es hat sich bei einer Betrachtung über die Wirtschaftlichkeit einer Eindampfung der niedrig-konzentrierten Lösung und einer Rückspeisung des Konzentrates herausgestellt, dass dieses Verfahren gegenüber einer Entsorgung als Abfall Kostenvorteile bietet. Dieser Vorteil erhöht sich auch dadurch, dass die in der Aufkonzentration des Caprolactam-Wassergemisches anfallenden Brüden 7 und die darin enthaltene Exergie wirtschaftlich genutzt wird. Dies geschieht nach dem vorliegenden Verfahren in dem diese Brüden 7 vorteilhaft wie in Abbildung 1 gezeigt, dem Sumpfreboiler W03 der Kolonne C02 zur Erhitzung und der Destillation des Extraktwassers zugeführt werden. Ein Teil des Kondensates 8 aus W03 wird nach Abgabe seines Wärmeinhaltes wieder als Rücklauf in die Aufkonzentration des Caprolactam in den Kopf von C01 eingespeist und der Rest im Neutralisationsbehälter R03 zur Bereitung der Neutralisationslösung und Quenchung des Rückstandstromes 3 eingesetzt.

Es hat sich überraschenderweise während des Betriebes einer Aufbereitungsanlage von Polyamid 6 Produkten herausgestellt, dass es wirtschaftlich und für die Produktreinheit des Caprolactam von Vorteil ist, wenn das Konzentrat 32 aus der Extraktions- und Eindickungskolonne dem Depolymerisationsreaktor R01 zugeführt wird.

Da die mechanische Aufbereitung und Trennung nach der Schwimm-Sink-Zentrifugen-Technologie nicht hundertprozentig reines Polyamid 6 und von Störstoffen vollständig befreites Polymer dem Extruder E01 anliefert in dem beispielsweise noch Anteile an SBR-Schaumrücken und Kreide aus der Aufbereitung von Teppichen, Russ, Geweberückstände sowie andere Füllstoffe im Stoffstrom 2 enthalten sind. Es hat sich beim kommerziellen Betrieb einer Caprolactam-Rückgewinnungsanlage als vorteilhaft erwiesen, diese Nebenbestandteile kontinuierlich und durch Analysen und online Messungen des Phosphorsäuregehaltes gesteuert über die Rückstandsleitung 3 auszuschleusen. Besonders vorteilhaft hat sich das Überwachen und Steuern der auszuschleusenden Masse durch Analysen und online Messungen des Phosphorsäuregehaltes in Strom im Hinblick auf den wirtschaftlichen Betrieb einer solchen Anlage erwiesen, da dadurch zum einen der Verbrauch an Phosphorsäure und zum anderen der Verbrauch an Alkali zur Neutralisation des Rückstandes 3 in Reaktor R03 minimiert werden konnte. Femer konnten durch Messung und Überwachung des Stickstoffgehaltes im auszuschleusenden Stoffstrom 3 vorteilhaft die Polymerkettenspaltung und somit die Ausbeute an Caprolactam gegenüber einer nicht Stickstoff überwachten Betriebsweise im Bereich von 5% -25 % gesteigert werden. Das vorstehend beschriebene hat sich als überaus vorteilhaft bei auf den Betrieb einer mit Polyamid 6 Abfällen betriebenen Caprolactamaufbereitungsanlage ausgewirkt. Im Gegensatz zu einer Caprolactamproduktion aus reinen Rohstoffen bei der es zu keiner Störung des kontinuierlichen Betriebes durch schwankende Zusammensetzung des eingehenden Produktstromes kommt, können die aus der wechselnden Zusammensetzung des Eingangsmaterials resultierenden Produktqualitätsschwankungen ausgeregelt und ausgeglichen werden.

Das in die Kolonne C01 gelangte Caprolactam-Wasser-Gemisch wird durch fraktionierte Destillation aufkonzentriert, wobei es sich als vorteilhaft erwiesen hat, dass durch Auswahl der Kolonneneinbauten, des Rücklaufverhältnisses im Bereich von 1:10 bis 10:1, besonders vorteilhaft 1: 2 bis 1:4, einer Temperatur im Bereich von 150°C bis 300°C und 0,1 bar bis 1 bar die im Caprolactamsumpf verbleibenden Nebenprodukte aus den Störstoffbeimengungen zu minimieren. Es hat sich im Hinblick auf die Reinheit des Caprolactam als vorteilhaft erwiesen, diese Nebenbestandteile kontinuierlich und durch Analysen und online Messungen zu überwachen und die Betriebsbedingungen wie beispielsweise Temperatur, Druck und Rücklaufverhältnis so zu steuern, dass die bestmögliche Entfernung der Nebenprodukte erreicht wird. Dies Ziel wird unter anderem dadurch erreicht, dass über einen Betriebszeitraum von 1000 Stunden verschiedene Parameter eingestellt werden und die online gemessenen Werte einen Mikrocomputer zur Auswertung mit einem Neuronalen Netzwerk Programm übergeben werden.

Das die Kolonne C01 verlassende Produkt 9 enthält noch in der wässrigen Phase dispergierte Stoffe. Es hat sich als vorteilhaft herausgestellt, für die Erreichung eines Rohcaprolactam mit guten Produkteigenschaften einen Abscheider A01 in die Leitung 10 einzubinden, in dem sich die in der wässrigen Phase dispergierten flüssigen, mitunter öligen Beimischungen abscheiden und als Stoffstrom 11 einer weiteren Verwertung wie z.B. Verbrennung zugeführt werden. Des weiteren konnte im Laufe des Anlagenbetriebs herausgefunden werden, dass ein Überleiten des Rohcaprolactamstromes 12 durch einen Festbettwechselfilter A02 sich vorteilhaft auf die Farbreinheit auswirkt.

In dem nachfolgenden Reaktor R02 findet die von der kommerziellen Caprolactamherstellung aus reinen Rohstoffen bekannte Behandlung der wässrigen Lösung mit Additiven und Oxidationsmitteln 14, so beispielsweise Kaliumpermanganat zur Verbesserung der Farbe, der Säurezahl und des Doppelbindungsgehaltes statt. Im Gegensatz zur industriellen Produktion die kontinuierlich und nur geringfügig schwankende Caprolactamqualität zu beherrschen hat und mit festeingestellten Additivzugaben arbeiten kann, schwanken die Produkteigenschaften der Caprolactamlösung aufgrund der sich mitunter extrem ändernden Zusammensetzung des eingehenden Polyamids. Um diesem Verhalten Rechnung zu tragen werden die Messergebnisse der Qualitätsüberwachung, die beispielsweise durch FTIR, Refraktometer, UV-Spektrometer erfolgen, an der Kolonne C01 benutzt, die Additivart und Additivzugabemasse so zu dosieren, dass ein geringst notwendiger Chemikalienverbrauch eine bestmögliche Rohcaprolactamqualität ergibt. Nach Abtrennung des Feststoffanteils im Abscheider A03, geht das Produkt 17 nochmals durch eine Festbettwechselfilter A04, was sich vorteilhaft auf die Farbreinheit auswirkt. Zur weiteren Konzentration der wässrigen Caprolactamlösung erfolgt die Einspeisung des Stromes 18 auf eine mittlere Trennstufe einer Rektifikationskolonne C03. Hier wird nach bekannten Verfahren Wasserdampf bei Drücken im Bereich von 20 mbar bis 250 mbar und Temperaturen im Bereich von 100°C bis 200 °C, Rücklaufverhältnis im Bereich von 1:10 bis 2:1 über Kopf abgezogen und es findet eine Aufkonzentrierung des Caprolactam unter Zugabe von alkalischen Neutralisationsmitteln 19 statt. Zur optimalen Nutzung von Abwärmen und dem Verbrauch an Wasser zur Spaltung des Polyamides hat es sich als vorteilhaft erwiesen, die Brüden 20 aus C03 nur teilweise und in der Menge zu kondensieren, dass die Rücklaufmenge gewährleistet ist. Die übrigen nicht kondensierten und heißen Dämpfe 20 werden nach Überhitzung in W04 dem Stoffstrom 4 zugesetzt.

Das den Sumpf der Kolonne C03 verlassende Konzentrat 21 wird in den Mittelteil einer weiteren Refraktionierungskolonne C04 aufgegeben, wo unter Drücken im Bereich von 0,5 mbar bis 50 mbar und Temperaturen im Bereich von 115°C bis 200 °C, Rücklaufverhältnis im Bereich von 1:10 bis 2:1 schwerer als Wasser siedende Komponenten abgetrennt werden. Es hat sich überraschenderweise herausgestellt, dass sich die Produkteigenschaften des zurückgewonnen Caprolactam entscheidend verbessern, wenn im unteren Teil der Kolonne ein Stickstoffstrom 23 zugeführt wird, der sowohl 100 ppm bis 5% Ammoniak beinhalten kann. Es hat beim Betrieb einer Teppichrecyclinganlage als vorteilhaft für die Erreichung eines niedrigen Säuregehaltes und einer guten Farbreinheit im Stoffstrom 24 erwiesen, bei der Rektifikation mit ammoniakhaltigem Stickstoff zu strippen.

In der Stufe C05, einer weiteren Rektifikationskolonne bei der eine wasserfreie Caprolactamschmelze in den Mittelteil eingespeist wird unter den dem Fachmann bekannten Bedingungen mit reduziertem Druck im Bereich von 0,5 mbar bis 50 mbar und Temperaturen im Bereich von 115°C bis 200 °C, Rücklaufverhältnis im Bereich von 1:10 bis 2:1 Caprolactam destilliert.

Im Sumpf der Kolonne bleiben Oligomere mit niedrigem Molekulargewicht, die sich mit wirtschaftlichem Vorteil noch zu Caprolactam spalten lassen und vorteilhaft auf die Ausbeute als Rückstandsstrom 25 zur erneuten Aufbereitung in den Extruder E01 oder den Reaktor R01 zurückgeführt werden.

Der Caprolactamdampf 26 wird in der Reinigungsstufe K01 durch Abkühlen partiell niedergeschlagen. Entsprechend dem Phasengleichgewicht weist der zuerst niedergeschlagene Feststoff einen höheren Reinheitsgrad auf als die zurück bleibende flüssige Restschmelze.

Diese Art der Reinigung von Caprolactam in K01 und K02 zeichnet sich dadurch aus, dass sie in zwei Schritten abläuft und sich an die bekannten Zonen- und Abschmelztechnologien anlehnt. Es hat sich als vorteilhaft herausgestellt, diese Kristallisation in einem Röhrenfallfilmapparat durchzuführen wie er unter anderem von der Fa. Sulzer bekannt ist. Der beim bekannten fraktionierten Kristallisieren auf die Abscheidung von Kristallen an einer Wand folgende Aufschmelzvorgang liefert ein zuerst anfallendes Produkt, das über die Leitung 27 der Kristallisationsstufe K02 zugeführt wird. Verbunden mit dieser Stufe ist der Analysator S01. Dieser Analysator S01erfüllt, so hat es sich vorteilhaft im Laufe des Betriebes herausgestellt, die Aufgabe das abgeschmolzene Produkt auf seine optischen Eigenschaften hin zu prüfen und je nach Güte die Schmelze diese über das Ventil V01 entweder dem Depolymerisationsreaktor R01, der Kolonne C04 oder C05 zu zuteilen oder bei entsprechender Reinheit ein zweites mal der Kristallisation K01 zuzuleiten. Der Ablauf des fraktionierten Kristallisations- und Abschmelzverfahrens ist bestimmt von der Schmelztemperatur der Caprolactamkristalle. Hochreines Caprolactam weist den höchsten Schmelzpunkt auf und wird zuerst an den Apparatewänden niedergeschlagen, die nachfolgenden Kristallschichten sind leicht mit Mutterlauge verunreinigt und kristallisieren später. Die am stärksten verschmutzten Schichten liegen aussen. Nach Unterschreiten der Temperatur in der Kristallschicht von beispielsweise 5°C unter den Schmelzpunkt von reinem Caprolactam wird der Durchfluss an Mutterlauge durch den Kristallisator gestoppt und es erfolgt die Umschaltung von Kühlen auf Heizen. Da die verunreinigten Kristalle zuerst schmelzen, tropfen diese ab, werden gesammelt und der zweiten Stufe K02 zugeführt. Nach beispielsweise dem Erreichen einer Temperatur von 2°C unter dem Schmelzpunkt von reinem Caprolactam in K01, wird der Abfluss zu K02 gestoppt, die Aufheizgeschwindigkeit erhöht und das reine Caprolactam abgeschmolzen. Dieser Vorgang aus K01 bekannte Vorgang wiederholt sich in K02 erneut, mit dem Unterschied, dass die zu Anfang abschmelzenden Kristalle abhängig von der Temperatur in andere Apparate und das Hauptprodukt der Kristallisation K02 zurück in die Kristallisation K01 verbracht werden . Erfindungsgemäß wird das zu Beginn des Abschmelzvorgangs stärker verschmutzte Produkt dem Depolymerisationsreaktor R01, das Produkt mit mittlerer Verschmutzung der Kolonne C03 und das zuletzt anfallende Produkt der Kolonne C04 oder der Reinigungsstufe K01 zugeleitet. Über die Mengen entscheidet das Analysenergebnis und ein Mikroprozessor gesteuertes Auswerte- und Regelsystem. Nachdem das Zonenabschmelzen in K01 von unreinem Produkt abgeschlossen ist, erfolgt das Abschmelzen des verbliebenen reinen Kristallisats 25, das gelagert bzw. gepuffert wird für die Abnahme durch eine Polykondensationsanlage. Dieser Puffer ist vorteilhaft und erfindungsgemäß ebenfalls mit einer Analysenvorrichtung versehen, wie sie zur Aufteilung des Vorschmelzproduktes 21 verwendet wird. Sie dient der Überwachung der Säurezahl und Farbreinheit und regelt die Freigabe des Produktes zur Weiterverarbeitung in einer Polykondensationsanlage. Sollte die Reinheit beispielweise durch zu lange Lagerung nicht mehr erreicht werden, so steuert dieser Analysator eine Rückführung des gesamten oder nur eines Teils des Inhaltes zur Reinigungsstufe K01/K02.

Der in der Depolymerisation R01 anfallende flüssige Strom 3 enthält anorganische Salze, überschüssige Phosphorsäure, artfremde Polymere und Oligomere, die nicht mehr zu Caprolactam gespalten werden können. Da das Produkt betriebsbedingt mehr Säure als ein neutrales Gemisch enthält, wird es vereinigt mit den Rückständen 12 aus dem Reaktor R02 vermischt und einer Neutralisationsstufe R03 zugeführt. Hier erfolgt die Zugabe von soviel Base, dass ein neutrales Salz 33 gebildet wird, das aufgrund seines organischen Restgehaltes einer thermischen Verwertung zugeführt wird.

### Beispiel 1:

Betrieb einer Produktionsanlage nach Verfahren nach Abbildung 1 und 2 mit faserigem, aus Teppichen zurückgewonnenem Polyamid 6
- Ohne Rückführung von Strom 25 Rückstand aus Kolonne C04,
- ohne kontinuierliche Phosphorsäure und Stickstoffüberwachung des Produktstromes 3 aus dem Depolymerisationsbehälter R01,
- ohne Kreislaufführung der Brüden 7 aus Kolonne C01 über W03 und Energieaustausch Polyamidextraktwasser C02,
- ohne Überwachung der Produkteigenschaften 9 nach C02 und keine Rückführregelung der Bedingungen in C02,
- ohne Feststofffiltration A02 und A03,
- ohne Rückführung der Brüden 20 aus Kolonne C03 und Nutzung als Dampf in R01
- ohne Einspeisung von ammoniakhaltiges Strippgas 23 in Kolonne C04
- ohne Qualitätsanalyse S01 im Abschmelzprodukt 27 der fraktionierten Kristallisation K01 und Steuerung der Rückführmengen durch V01 zu E01, R01, C04 und C05

### Beispiel 2:

Betrieb einer Produktionsanlage nach Verfahren nach Beispiel 1 nach Einbau
- Rückführung von Strom 25 Rückstand aus Kolonne C04,
- kontinuierlicher Phosphorsäure und Stickstoffüberwachung des Produktstromes 3 aus dem Depolymerisationsbehälter R01,

### Beispiel 3:

Betrieb einer Produktionsanlage nach Verfahren nach Beispiel 2 nach Einbau
- Kreislaufführung der Brüden 7 aus Kolonne C01 über W03 und Energieaustausch Polyamidextraktwasser C02,
- Überwachung der Produkteigenschaften 9 nach C02 und keine Rückführregelung der Bedingungen in C02,
- Feststofffiltration A02 und A03,

### Beispiel 4:

Betrieb einer Produktionsanlage nach Verfahren nach Beispiel 3 nach Einbau von
- Rückführung der Brüden 20 aus Kolonne C03 und Nutzung als Dampf in R01
- Einspeisung von ammoniakhaltiges Strippgas 23 in Kolonne C04
- Qualitätsanalysegerät S01 im Abschmelzprodukt 27 der fraktionierten Kristallisation K01 und Steuerung der Rückführmengen durch V01 zu E01, R01, C04 und C05

### Beispiel 5:

Wie Beispiel 4, geänderter Rohstoff
Gewölleabfälle aus der Produktion von Polyamidfasern

**Tabelle 1**

| **Verfahren** | **Rohstoff** | **Polyamid 6 Gehalt Massen%** | **Nebenprodukte Im Rohstoff** | **Ausbeute Caprolactam %** | **Permanganat-Zahl sec** | **UV-Transmission %** | **Relativer Energieverbrauch %** |
|---|---|---|---|---|---|---|---|
| industriell | Caprolactam | - | - | - | >10000 | >85 | - |
| Beispiel 1 * | PA6 Faser Teppich | 90 | Kreide, SBR,PP | 85% | Min. 5000 Max. 8000 | Min. 75 Max. 85 | 100 |
| Beispiel 2 * | PA6 Faser Teppich | 90 | Kreide, SBR,PP | 86 | Min. 6500 Max. 8000 | Min. 80 Max. 85 | 95 |
| Beispiel 3 * | PA6 Faser Teppich | 90 | Kreide, SBR,PP | 90 | Min. 10000 Max. 12000 | Min. 85 Max. 90 | 90 |
| Beispiel 4 | PA6 Faser Teppich | 75-96 | Kreide, SBR,PP | 91 | Min. 25000 Max. 27000 | Min. 88 Max. 90 | 80 |
| Beispiel 5 | PA6 Faser Gewölle | 99 | Öle, Appreturen | 97 | 26000 | 89 | 65 |

| | | | | | | | |
|---|---|---|---|---|---|---|---|
| * nicht erfindungsgemäß | | | | | | | |

## Patentansprüche

1. Verfahren zur Herstellung von Caprolactam aus Polyamid-haltigen Abfällen, umfassend die Schritte
a) Depolymerisieren der Polyamid-haltigen Abfälle, wodurch ein Caprolactam-Rohmaterial und gegebenenfalls ein Nebenbestandteile bzw. Zuschlagsstoffe enthaltender Strom erhalten wird,
b) wenigstens einmaliges Destillieren des Caprolactam-Rohmaterials, und
c) wenigstens einmaliges Kristallisieren des in Stufe b) erhaltenen Caprolactam-Materials, wodurch Caprolactam erhalten wird,
wobei wenigstens ein Teil des in Stufe c) erhaltenen Caprolactams mit einer Permanganatzahl von < 10000 sec. und einer UV-Transmission von < 85 % den Polyamid-haltigen Abfällen vor und/oder während der Depolymerisation zugesetzt wird.

2. Verfahren nach Anspruch 1, wobei wenigstens ein Teil des in Stufe c) erhaltenen Caprolactams mit einer Permanganatzahl von < 10000 sec. und einer UV-Transmission von < 85 % in Stufe b) zugesetzt wird.

3. Verfahren nach einem der vorstehenden Ansprüche, wobei dieses kontinuierlich durchgeführt wird.

4. Verfahren nach einem der vorstehenden Ansprüche, wobei in Stufe a) ein saurer Katalysator zugesetzt wird.

5. Verfahren nach einem der vorstehenden Ansprüche, wobei Nebenbestandteile bzw. Zuschlagsstoffe kontinuierlich und durch Analyse und Überwachung des Spaltsäure- und Stickstoffgehalts des Stromes, der die Nebenbestandteile bzw. Zuschlagsstoffe enthält und in Stufe a) erhalten wird, kontrolliert und geregelt ausgeschleust wird.

6. Verfahren nach Anspruch 5, wobei die Analyse und Überwachung kontinuierlich durchgeführt wird.

7. Verfahren nach einem der vorstehenden Ansprüche, wobei die Reaktionsbedingungen in Stufe a) der sich ändemden Zusammensetzung des Polyamid-haltigen Materials angepasst werden.

8. Verfahren nach einem der vorstehenden Ansprüche, wobei das nach der Stufe b) erhaltene Caprolactam durch kontinuierliche Analysen auf seine Qualitätsmerkmale, wie Permanganatzahl, UV-transmission, APHA-Zahl, Alkalität und flüchtigen Basengehalt, untersucht wird.

9. Verfahren nach einem der vorstehenden Ansprüche, wobei das nach Stufe b) erhaltene Caprolactam zur Verbesserung der Qualitätsmerkmale, wie Permanganatzahl, UV-Transmission, APHA-Zahl, Alkalität und flüchtigen Basengehalt, durch wenigstens einen Abscheider geleitet wird.

10. Verfahren nach einem der vorstehenden Ansprüche, wobei dem nach Stufe b) erhaltenen Caprolactam Additive und/oder Oxidationsmittel zugesetzt wird.

11. Verfahren nach einem der vorstehenden Ansprüche, wobei in Stufe b) mehr als eine Destillation durchgeführt wird und dem nach der ersten und vor der letzten Destillation erhaltenen Caprolactam mindestens einmal Additive und/oder Oxidationsmittel zugesetzt wird.

12. Verfahren nach einem der vorstehenden Ansprüche, wobei zusätzlich vor und/oder während der Stufe b) eine Festbettfiltration durchgeführt wird.

13. Verfahren nach Anspruch 12, wobei dem nach und/oder während der Stufe b) erhaltenem Caprolactam Additive und/oder Oxidationsmittel zugesetzt werden und vor und/oder nach deren Zugabe eine Festbettfiltration durchgeführt wird.

## Claims

1. A method for the manufacture of caprolactam from polyamide-containing waste, comprising the steps of
a) depolymerising the polyamide-containing waste, whereby a caprolactam raw material and, where applicable, a flow containing secondary constituents or additives is obtained,
b) distilling the caprolactam raw material at least once, and
c) crystallising at least once the caprolactam material obtained in step b), whereby caprolactam is obtained,
wherein at least part of the caprolactam obtained in step c) with a permanganate number of < 10000 sec. and a UV transmission of < 85% is added to the polyamide-containing waste before and/or during the depolymerisation.

2. The method according to claim 1, wherein at least part of the caprolactam obtained in step c) with a permanganate number of < 10000 sec. and a UV transmission of < 85% is added to step b).

3. The method according to one of the preceding claims, wherein it is carried out continuously.

4. The method according to one of the preceding claims, wherein an acidic catalyst is added in step a).

5. The method according to one of the preceding claims, wherein secondary constituents or additives are continuously and through analysis and monitoring of the cracking acid and nitrogen content of the flow, which contains the secondary constituents or additives and is obtained in step a), discharged in a controlled manner.

6. The method according to claim 5, whereby the analysis and monitoring are carried out continuously.

7. The method according to one of the preceding claims, whereby the reaction conditions in step a) are adapted to the changing composition of the polyamide-containing material.

8. The method according to one of the preceding claims, wherein the caprolactam obtained after step b) is examined through continuous analysis for its quality features, such as permanganate number, UV transmission, APHA number, alkalinity and volatile base content.

9. The method according to one of the preceding claims, wherein the caprolactam obtained after step b) is passed through at least one separator to improve the quality features, such as permanganate number, UV transmission, APHA number, alkalinity and volatile base content.

10. The method according to one of the preceding claims, wherein additives and/or oxidation agents are added to the caprolactam obtained after step b).

11. The method according to one of the preceding claims, wherein in step b) more than one distillation is carried out and additives and/or oxidation agents are added at least once to the caprolactam obtained after the first and before the last distillation.

12. The method according to one of the preceding claims, wherein in addition a packed bed filtration is carried out before and/or during step b).

13. The method according to claim 12, wherein additives and/or oxidation agents are added to the caprolactam obtained after and/or during step b) and a packed bed filtration is carried out before and/or after their addition.

## Revendications

1. Procédé de préparation du caprolactame à partir de déchets contenant des polyamides, comprenant les étapes :
a) une dépolymérisation des déchets contenant les polyamides, par laquelle est obtenu un matériau brut de caprolactame et, le cas échéant, un courant contenant des composants secondaires, respectivement des additifs,
b) au moins une passe de distillation du matériau brut de caprolactame et
c) au moins une passe de cristallisation du matériau de caprolactame obtenu à l'étape b), par laquelle est obtenu le caprolactame,
sachant qu'au moins une partie du caprolactame obtenu à l'étape c), avec un indice de permanganate de <10000 sec. et une transmission des UV de <85 %, est ajouté avant et/ou pendant la dépolymérisation aux déchets contenant des polyamides.

2. Procédé selon la revendication 1, dans lequel au moins une partie du caprolactame obtenu à l'étape c), avec un indice de permanganate de <10000 et une transmission des UV de <85 %, est ajouté dans l'étape b).

3. Procédé selon l'une des revendications précédentes, sachant que celui-ci est effectué en continu.

4. Procédé selon l'une des revendications précédentes, dans lequel un catalyseur acide est ajouté à l'étape a).

5. Procédé selon l'une des revendications précédentes, dans lequel des composants secondaires, respectivement des additifs, sont soutirés de façon contrôlée et régulée, continûment et par analyse et surveillance de la teneur en acides de craquage et en azote du courant qui contient les composants secondaires, respectivement les additifs et qui est obtenu à l'étape a).

6. Procédé selon la revendication 5, dans lequel l'analyse et la surveillance sont effectuées en continu.

7. Procédé selon l'une des revendications précédentes, dans lequel les conditions de réaction à l'étape a) sont adaptées à la composition variable du matériau contenant les polyamides.

8. Procédé selon l'une des revendications précédentes, dans lequel le caprolactame obtenu après l'étape b) est étudié par analyse continue quant à ses caractéristiques de qualité comme l'indice de permanganate, la transmission des UV, l'indice APHA, la basicité et le contenu en bases volatiles.

9. Procédé selon l'une des revendications précédentes, dans lequel le caprolactame obtenu après l'étape b) est envoyé à travers au moins un séparateur pour l'amélioration des caractéristiques de qualité comme l'indice de permanganate, la transmission des UV, l'indice APHA, la basicité et le contenu en bases volatiles.

10. Procédé selon l'une des revendications précédentes, dans lequel on ajoute au caprolactame obtenu après l'étape b) des additifs et/ou des agents oxydants.

11. Procédé selon l'une des revendications précédentes, dans lequel plus d'une distillation est effectuée à l'étape b) et dans lequel, après la première et avant la dernière distillation, des additifs et/ou des agents oxydants sont ajoutés au moins une fois au caprolactame obtenu.

12. Procédé selon l'une des revendications précédentes, dans lequel une filtration sur lit fixe est effectuée en plus, avant et/ou pendant l'étape b).

13. Procédé selon la revendication 12, dans lequel des additifs et/ou des agents oxydants sont ajoutés au caprolactame obtenu avant et/ou pendant l'étape b), et dans lequel une filtration sur lit fixe est effectuée avant et/ou après leur addition.
